# EUROPEAN PATENT SPECIFICATION

(11) **EP 1 971 338 B1**
(45) Date of publication and mention of the grant of the patent: **09.03.2011**
(21) Application number: 06820566.5
(22) Date of filing: 19.12.2006
(51) Int. Cl.: A61K 31/517, A61K 31/519, A61P 35/00

(54) **COMBINATION OF ZD6474 AND PEMETREXED**
KOMBINATION VON ZD6474 UND PEMETREXED
COMBINAISON DE ZD6474 ET DE PEMETREXED

(30) Priority: 22.12.2005 GB 0526121; 31.05.2006 GB 0610706
(43) Date of publication of application: 24.09.2008
(73) Proprietor: AstraZeneca AB, 151 85 Södertälje (SE)
(72) Inventor: WEDGE, Stephen, Robert, Cheshire SK10 4TG (GB); RYAN, Anderson, Joseph, Cheshire SK10 4TG (GB)
(86) International application number: PCT/GB2006/004750
(87) International publication number: WO 2007/071958

(56) References cited:
- HUBER PETER E ET AL: "Trimodal cancer treatment: Beneficial effects of combined antianglogenesis, radiation, and chemotherapy" CANCER RESEARCH, vol. 65, no. 9, May 2005 (2005-05), pages 3643-3655,3633, XP002464232 ISSN: 0008-5472
- BISCHOF M ET AL: "Triple combination of irradiation, chemotherapy (pemetrexed), and VEGFR inhibition (SU5416) in human endothelial and tumor cells" INTERNATIONAL JOURNAL OF RADIATION: ONCOLOGY BIOLOGY PHYSICS, PERGAMON PRESS, US, vol. 60, no. 4, 15 November 2004 (2004-11-15), pages 1220-1232, XP004618608 ISSN: 0360-3016
- GLADE-BENDER J ET AL: "VEGF BLOCKING THERAPY IN THE TREATMENT OF CANCER" EXPERT OPINION ON BIOLOGICAL THERAPY, ASHLEY, LONDON, GB, vol. 3, no. 2, April 2003 (2003-04), pages 263-276, XP009060849 ISSN: 1471-2598
- SRIDHAR SRIKALA S ET AL: "Targeting angiogenesis: a review of angiogenesis inhibitors in the treatment of lung cancer." LUNG CANCER (AMSTERDAM, NETHERLANDS) DEC 2003, vol. 42 Suppl 1, December 2003 (2003-12), pages S81-S91, XP002464233 ISSN: 0169-5002

## Description

The present invention relates to the treatment of a cancer, particularly a cancer involving a solid tumour, by the administration of ZD6474 in combination with pemetrexed; to a pharmaceutical composition comprising ZD6474 and pemetrexed; to a combination product comprising ZD6474 and pemetrexed for use in a treatment of a human or animal body by therapy; to a kit comprising ZD6474 and pemetrexed; to the use ofZD6474 and pemetrexed in the manufacture of a medicament for use in the production of an anti-cancer effect in a warm-blooded animal such as a human which is optionally being treated with ionising radiation.

Normal angiogenesis plays an important role in a variety of processes including embryonic development, wound healing and several components of female reproductive function. Undesirable or pathological angiogenesis has been associated with disease states including diabetic retinopathy, psoriasis, cancer, rheumatoid arthritis, atheroma, Kaposi's sarcoma and haemangioma (Fan et al, 1995, Trends Pharmacol. Sci. 16: 57-66; Folkman, 1995, Nature Medicine 1: 27-31). Alteration of vascular permeability is thought to play a role in both normal and pathological physiological processes (Cullinan-Bove et al, 1993, Endocrinology 133: 829-837; Senger et al, 1993, Cancer and Metastasis Reviews, 12: 303-324). Several polypeptides with *in vitro* endothelial cell growth promoting activity have been identified including, acidic and basic fibroblast growth factors (aFGF & bFGF) and vascular endothelial growth factor (VEGF). By virtue of the restricted expression of its receptors, the growth factor activity of VEGF, in contrast to that of the FGFs, is relatively specific towards endothelial cells. Recent evidence indicates that VEGF is an important stimulator of both normal and pathological angiogenesis (Jakeman et al, 1993, Endocrinology, 133: 848-859; Kolch et al, 1995, Breast Cancer Research and Treatment, 36; 19-155) and vascular permeability (Connolly et al, 1989, J. Biol. Chem. 264: 20017-20024). Antagonism of VEGF action by sequestration of VEGF with antibody can result in inhibition of tumour growth (Kim et al, 1993, Nature 362: 841-844).

Receptor tyrosine kinases (RTKs) are important in the transmission of biochemical signals across the plasma membrane of cells. These transmembrane molecules characteristically consist of an extracellular ligand-binding domain connected through a segment in the plasma membrane to an intracellular tyrosine kinase domain. Binding of ligand to the receptor results in stimulation of the receptor-associated tyrosine kinase activity which leads to phosphorylation of tyrosine residues on both the receptor and other intracellular molecules. These changes in tyrosine phosphorylation initiate a signalling cascade leading to a variety of cellular responses. To date, at least nineteen distinct RTK subfamilies, defined by amino acid sequence homology, have been identified. One of these subfamilies is presently comprised by the fms-like tyrosine kinase receptor, Flt-1 (also referred to as VEGFR-1), the kinase insert domain-containing receptor, KDR (also referred to as VEGFR-2 or Flk-1), and another fms-like tyrosine kinase receptor, Flt-4. Two of these related RTKs, Flt-1 and KDR, have been shown to bind VEGF with high affinity (De Vries et al, 1992, Science 255: 989-991; Terman et al, 1992, Biochem. Biophys. Res. Comm. 1992, 187: 1579-1586). Binding of VEGF to these receptors expressed in heterologous cells has been associated with changes in the tyrosine phosphorylation status of cellular proteins and calcium fluxes.

VEGF is a key stimulus for vasculogenesis and angiogenesis. This cytokine induces a vascular sprouting phenotype by inducing endothelial cell proliferation, protease expression and migration, and subsequent organisation of cells to form a capillary tube (Keck, P.J., Hauser, S.D., Krivi, G., Sanzo, K., Warren, T., Feder, J., and Connolly, D.T., Science. (Washington DC), 246: 1309-1312, 1989; Lamoreaux, W.J., Fitzgerald, M.E., Reiner, A., Hasty, K.A., and Charles, S.T., Microvasc. Res., 55: 29-42, 1998; Pepper, M.S., Montesano, R., Mandroita, S.J., Orci, L. and Vassalli, J.D., Enzyme Protein, 49: 138-162, 1996.). In addition, VEGF induces significant vascular permeability (Dvorak, H.F., Detmar, M., Claffey, K.P., Nagy, J.A., van de Water, L., and Senger, D.R., (Int. Arch. Allergy Immunol., 107: 233-235, 1995; Bates, D.O., Heald, R.I., Curry, F.E. and Williams, B. J. Physiol. (Lond.), 533: 263-272, 2001), promoting formation of a hyper-permeable, immature vascular network which is characteristic of pathological angiogenesis.

It has been shown that activation of KDR alone is sufficient to promote all of the major phenotypic responses to VEGF, including endothelial cell proliferation, migration, and survival, and the induction of vascular permeability (Meyer, M., Clauss, M., Lepple-Wienhues, A., Waltenberger, J., Augustin, H.G., Ziche, M., Lanz, C., Büttner, M., Rziha, H-J., and Dehio, C., EMBO J., 18: 363-374,1999; Zeng, H., Sanyal, S. and Mukhopadhyay, D., J. Biol. Chem., 276: 32714-32719,2001; Gille, H., Kowalski, J., Li, B., LeCouter, J., Moffat, B, Zioncheck, T.F., Pelletier, N. and Ferrara, N., J. Biol. Chem., 276: 3222-3230, 2001).

Quinazoline derivatives which are inhibitors of VEGF receptor tyrosine kinase are described in International Patent Applications Publication Nos. WO 98/13354 and WO 01/32651. In WO 98/13354 and WO 01/32651 compounds are described which possess activity against VEGF receptor tyrosine kinase (VEGF RTK) whilst possessing some activity against epidermal growth factor (EGF) receptor tyrosine kinase (EGF RTK). ZD6474 is 4-(4-bromo-2-fluoroanilino)-6-methoxy-7-(1-methylpiperidin-4-ylmethoxy)quinazoline:

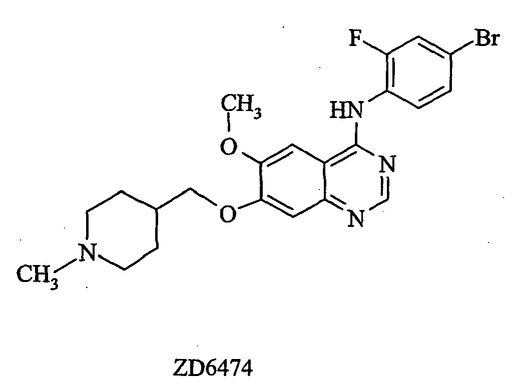

ZD6474 is also known as vandetanib and as ZACTIMA^{™} (trademark of the AstraZeneca group of companies).

ZD6474 falls within the broad general disclosure of WO 98/13354 and is exemplified in WO 01/32651. ZD6474 is a potent inhibitor of VEGF RTK and also has some activity against EGF RTK. ZD6474 has been shown to elicit broad-spectrum anti-tumour activity in a range of models following once-daily oral administration (Wedge SR, Ogilvie DJ, Dukes M, et al. ZD6474 inhibits vascular endothelial growth factor signaling, angiogenesis, and tumour growth following oral administration. Cancer Res 2002;62:4645-4655).

In WO 98/13354 and WO 01/32651 it is stated that compounds of their inventions: "may be applied as a sole therapy or may involve, in addition to a compound of the invention, one or more other substances and/or treatments. Such conjoint treatment may be achieved by way of the simultaneous, sequential or separate administration of the individual components of the treatment."

WO 98/13354 and WO 01/32651 then go on to describe examples of such conjoint treatment including surgery, radiotherapy and various types of chemotherapeutic agent.

Nowhere in WO 98/13354 and WO 01/32651 do they suggest the combination of a compound of the invention and pemetrexed for the treatment of any disease state including cancer.

Nowhere in WO 98/13354 and WO 01/32651 is the specific combination of ZD6474 and pemetrexed suggested.

Nowhere in WO 98/13354 and WO 01/32651 does it state that use of any compound of the invention therein with other treatments will produce surprisingly beneficial effects.

Pemetrexed is commonly used as pemetrexed disodium heptahydrate which has the chemical name:
L-Glutamic acid, *N*-[4-[2-(2-amino-4,7-dihydro-4-oxo-1*H*-pyrrolo[2,3-d]pyrimidin-5-yl)ethyl]benzoyl]-, disodium salt, hetpahydrate. The structural formula is as follows: 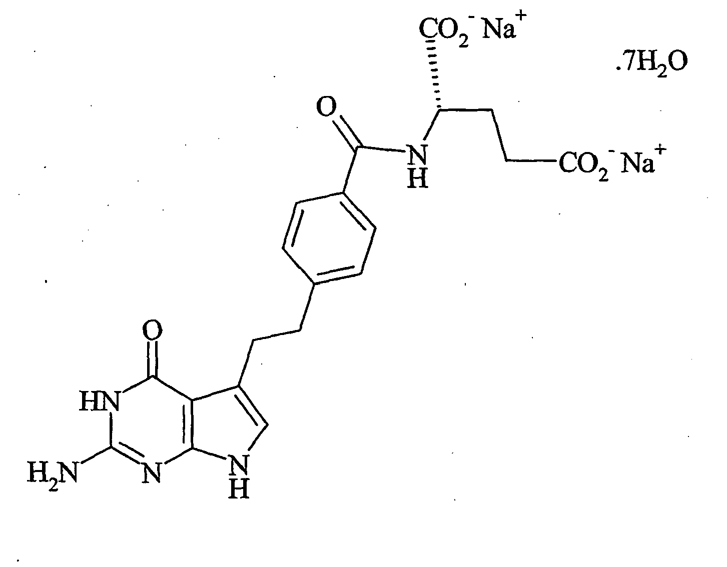
Pemetrexed is also known as ALIMTA™ (Trademark of Lilly) and it is an anti-cancer antifolate agent that disrupts folate-dependeut metabolic processes involved in cell replication.

Anti-cancer effects of the present invention include, but are not limited to, anti-tumour effects, the response rate, the time to disease progression and the survival rate. Anti-tumour effects of the present invention include but are not limited to, inhibition of tumour growth, tumour growth delay, regression of tumour, shrinkage of tumour, increased time to regrowth of tumour on cessation of treatment, slowing of disease progression. It is expected that when a combination treatment of the present invention is administered to a warm-blooded animal such as a human, in need of treatment for cancer, said combination treatment will produce an effect, as measured by, for example, one or more of: the extent of the anti-tumour effect, the response rate, the time to disease progression and the survival rate. Anti-cancer effects include prophylactic treatment as well as treatment of existing disease.

A method for the production of an antiangiogenic and/or vascular permeability reducing effect in a warm-blooded animal such as a human, may comprise administering to said animal an effective amount of ZD6474 or a pharmaceutically acceptable salt thereof, before, after or simultaneously with an effective amount of pemetrexed.

A method for the treatment of a cancer in a warm-blooded animal such as a human, may comprise administering to said animal an effective amount of ZD6474 or a pharmaceutically acceptable salt thereof, before, after or simultaneously with an effective amount of pemetrexed.

A method for the treatment of a cancer involving a solid tumour in a warm-blooded animal such as a human, may comprise administering to said animal an effective amount of ZD6474 or a pharmaceutically acceptable salt thereof, before, after or simultaneously with an effective amount of pemetrexed.

A method for the treatment of malignant pleural mesothelioma in a warm-blooded animal such as a human, may comprise administering to said animal an effective amount of ZD6474 or a pharmaceutically acceptable salt thereof, before, after or simultaneously with an effective amount of pemetrexed.

A method for the treatment of non-small cell lung cancer (NSCLC) in a warm-blooded animal such as a human, may comprise administering to said animal an effective amount of ZD6474 or a pharmaceutically acceptable salt thereof, before, after or simultaneously with an effective amount of pemetrexed.

A method for the treatment of small cell lung cancer (SCLC) in a warm-blooded animal such as a human, may comprise administering to said animal an effective amount of ZD6474 or a pharmaceutically acceptable salt thereof, before, after or simultaneously with an effective amount of permetrexed.

A method for the production of an antiangiogenic and/or vascular permeability reducing effect in a warm blooded animal such as a human, may comprise administering to said animal an effective amount of ZD6474 or a pharmaceutically acceptable salt thereof, before, after or simultaneously with an effective amount of pemetrexed wherein ZD6474 and pemetrexed may each optionally be administered together with a pharmaceutically acceptable excipient or carrier.

A method for the treatment of a cancer in a warm-blooded animal such as a human, may comprise administering to said animal an effective amount of ZD6474 or a pharmaceutically acceptable salt thereof, before, after or simultaneously with an effective amount of pemetrexed; wherein ZD6474 and pemetrexed may each optionally be administered together with a pharmaceutically acceptable excipient or carrier.

A method for the treatment of a cancer involving a solid tumour in a warm-blooded animal such as a human, may comprise administering to said animal an effective amount of ZD6474 or a pharmaceutically acceptable salt thereof, before, after or simultaneously with an effective amount of pemetrexed; wherein ZD6474 and pemetrexed may each optionally be administered together with a pharmaceutically acceptable excipient or carrier.

A method for the treatment of malignant pleural mesothelioma in a warm-blooded animal such as a human, may comprise administering to said animal an effective amount of ZD6474 or a pharmaceutically acceptable salt thereof before, after or simultaneously with an effective amount of pemetrexed; wherein ZD6474 and pemetrexed may each optionally be administered together with a pharmaceutically acceptable excipient or carrier.

A method for the treatment of non-small cell lung cancer (NSCLC) in a warm-blooded animal such as a human, may comprise administering to said animal an effective amount of ZD6474 or a pharmaceutically acceptable salt thereof, before, after or simultaneously with an effective amount of pemetrexed; wherein ZD6474 and pemetrexed may each optionally be administered together with a pharmaceutically acceptable excipient or carrier.

A method for the treatment of small cell lung cancer (SCLC) in a warm-blooded animal such as a human, may comprise administering to said animal an effective amount of ZD6474 or a pharmaceutically acceptable salt thereof, before, after or simultaneously with an effective amount of pemetrexed; wherein ZD6474 and pemetrexed may each optionally be administered together with a pharmaceutically acceptable excipient or carrier.

According to an aspect of the invention there is provided a pharmaceutical composition which comprises ZD6474 or a pharmaceutically acceptable salt thereof, and pemetrexed in association with a pharmaceutically acceptable excipient or carrier.

According to a further aspect of the present invention there is provided a combination product comprising ZD6474 or a pharmaceutically acceptable salt thereof and pemetrexed, for use in a method of treatment of a human or animal body by therapy.

According to a further aspect of the present invention there is provided a kit comprising ZD6474 or a pharmaceutically acceptable salt thereof, and pemetrexed

According to a further aspect of the present invention there is provided a kit comprising:
a) ZD6474 or a pharmaceutically acceptable salt thereof in a first unit dosage form;
b) pemetrexed in a second unit dosage form; and
c) container means for containing said first and second dosage forms.

According to a further aspect of the present invention there is provided a kit comprising:
a) ZD6474 or a pharmaceutically acceptable salt thereof, together with a pharmaceutically acceptable excipient or carrier, in a first unit dosage form;
b) pemetrexed together with a pharmaceutically acceptable excipient or carrier, in a second unit dosage form; and
c) container means for containing said first and second dosage forms.

There may be provided the use of ZD6474 or a pharmaceutically acceptable salt thereof and pemetrexed in the manufacture of a medicament for use in the production of an antiangiogenic and/or vascular permeability reducing effect in a warm-blooded animal such as a human.

According to a further aspect of the present invention there is provided the use of ZD6474 or a pharmaceutically acceptable salt thereof and pemetrexed in the manufacture of a medicament for use in the production of an anti-cancer effect in a warm-blooded animal such as a human.

According to a further aspect of the present invention there is provided the use of ZD6474 or a pharmaceutically acceptable salt thereof and pemetrexed in the manufacture of a medicament for use in the production of an anti-tumour effects in a warm-blooded animal such as a human.

According to a further aspect of the present invention there is provided the use of ZD6474 or a pharmaceutically acceptable salt thereof and pemetrexed in the manufacture of a medicament for use in the production of an anti-cancer effect in a warm-blooded animal such as a human wherein the cancer is malignant pleural mesothelioma.

According to a further aspect of the present invention there is provided the use of ZD6474 or a pharmaceutically acceptable salt thereof and pemetrexed in the manufacture of a medicament for use in the production af an anti-tumour effect in a warm-blooded animal such as a human wherein the tumour is a malignant pleural mesothelioma.

According to a further aspect of the present invention there is provided the use of ZD6474 or a pharmaceutically acceptable salt thereof and pemetrexed in the manufacture of a medicament for use in the production of an anti-cancer effects in a warm-blooded animal such as a human wherein the cancer is non-small cell lung cancer (NSCLC).

According to a further aspect of the present invention there is provided the use of ZD6474 or a pharmaceutically acceptable salt thereof and pemetrexed in the manufacture of a medicament for use in the production of an anti-tumour effect in a warm-blooded animal such as a human wherein the tumour is a non-small cell tumour of the lung.

According to a further aspect of the present invention there is provided the use of ZD6474 or a pharmaceutically acceptable salt thereof and pernetrexed in the manufacture of a medicament for use in the production of an anti-cancer effect in a warm-blooded animal such as a human wherein the cancer is small cell lung cancer (SCLC).

According to a further aspect of the present invention there is provided the use of ZD6474 or a pharmaceutically acceptable salt thereof and pemetrexed in the manufacture of a medicament for use in the production of an anti-tumour effect in a warm blooded animal such as a human wherein the tumour is a small cell tumour of the lung.

According to a further aspect of the present invention there is provided a combination treatment comprising the administration of an effective amount of ZD6474 or a pharmaceutically acceptable salt thereof, optionally together with a pharmaceutically acceptable excipient or carrier, and the simultaneous, sequential or separate administration of an effective amount of pemetrexed; wherein pemetrexed may optionally be administered together with a pharmaceutically acceptable excipient or carrier; to a warm-blooded animal such as a human in need of such therapeutic treatment

Such therapeutic treatment includes an anti-cancer effect and an anti-tumour effect.

A combination treatment of the present invention as defined herein may be achieved by way of the simultaneous, sequential or separate administration of the individual components of said treatments. A combination treatment as defined herein may be applied as a sole therapy or may involve surgery or radiotherapy or an additional chemotherapeutic agent in addition to a combination treatment of the invention.

Surgery may comprise the step of partial or complete tumour resection, prior to, during or after the administration of the combination treatment with ZD6474 described herein.

Other chemotherapeutic agents for optional use with a combination treatment of the present invention include those described in WO 01/32651.

Such chemotherapy may cover five main categories of therapeutic agent:
(i) other antiangiogenic agents including vascular targeting agents;
(ii) cytostatic agents;
(iii) biological response modifiers (for example interferon);
(iv) antibodies (for example edrecolomab); and
(v) antiproliferative/antineoplastic drugs and combinations thereof, as used in medical oncology; and other categories of agent are:
(vi) antisense therapies;
(vii) gene therapy approaches; and
(ix) immunotherapy approaches.
Particular examples of chemotherapeutic agents for use with a combination treatment of the present invention are raltitrexed, etoposide, vinorelbine, paclitaxel, docetaxel, cisplatin, oxaliplatin, carboplatin, gemcitabine, irinotecan (CPT- 11), 5 -fluorouracil (5-FU, (including capecitabine)), doxorubicin, cyclophosphamide, temozolomide and hydroxyurea. Such combinations are expected to be particularly useful for the treatment of cancer of the lung, head and neck, brain, colon, rectum, oesophagus, stomach, cervix, ovary, skin, breast, bladder, prostate, pancreas and including haematological malignancies. Such combinations are expected to be more particularly useful for the treament of cancer of the pancreas, colorectal cancer, malignant pleural mesothelioma, non-small cell lung cancer (NSCLC), breast cancer and bladder cancer.

The administration of a triple combination of ZD6474, pemetrexed and ionising radiation may produce effects, such as anti-tumour effects, greater than those achieved with any of ZD6474, pemetrexed and ionising radiations used alone, greater than those achieved with the combination of ZD6474 and pemetrexed, greater than those achieved with the combination of ZD6474 and ionising radiation, greater than those achieved with the combination of pemetrexed and ionising radiation.

A method for the production of an antiangiogenic and/or vascular permeability reducing effect in a warm-blooded animal such as a human, may comprise administering to said animal an effective amount of ZD6474 or a pharmaceutically acceptable salt thereof, before, after or simultaneously with an effective amount of pemetrexed and before, after or simultaneously with an effective amount of ionising radiation.

A method for the treatment of a cancer in a warm-blooded animal such as a human, may comprise administering to said animal an effective amount of D6474 or a pharmaceutically acceptable salt thereof, before, after or simultaneously with an effective amount of pemetrexed and before, after or simultaneously with an effective amount of ionising radiation.

A method for the treatment of a cancer involving a solid tumour in a warm-blooded animal such as a human, may comprise administering to said animal an effective amount of ZD6474 or a pharmaceutically acceptable salt thereof, before, after or simultaneously with an effective amount of pemetrexed and before, after or simultaneously with an effective amount of ionising radiation.

A method for the treatment of malignant pleural mesothelioma in a warm-blooded animal such as a human, may comprise administering to said animal an effective amount of ZD6474 or a pharmaceutically acceptable salt thereof, before, after or simultaneously with an effective amount of pemetrexed and before, after or simultaneously with an effective amount of ionising radiation.

A method for the treatment of non-small cell lung cancer (NSCLC) in a warm-blooded animal such as a human, may comprise administering to said animal an effective amount of ZD6474 or a pharmaceutically acceptable salt thereof, before, after or simultaneously with an effective amount of pemetrexed and before, after or simultaneously with an effective amount of ionising radiation.

A method for the treatment of small cell lung cancer (SCLC) in a warm-blooded animal such as a human, may comprise administering to said animal an effective amount of ZD6474 or a pharmaceutically acceptable salt thereof, before, after or simultaneously with an effective amount of pemetrexed and before, after or simultaneously with an effective amount of ionising radiation.

A method for the production of an antiangiogenic and/or vascular permeability reducing effect in a warm-blooded animal such as a human, may comprise administering to said animal an effective amount of ZD6474 or a pharmaceutically acceptable salt thereof, before, after or simultaneously with an effective amount of pemetrexed and before, after or simultaneously with an effective amount of ionising radiation, wherein ZD6474 and pemetrexed may each optionally be administered together with a pharmaceutically acceptable excipient or carrier.

A method for the treatment of a cancer in a warm-blooded animal such as a human, may comprise administering to said animal an effective amount of ZD6474 or a pharmaceutically acceptable salt thereof, before, after or simultaneously with an effective amount of pemetrexed and before, after or simultaneously with an effective amount of ionising radiation, wherein ZD6474 and pemetrexed may each optionally be administered together with a pharmaceutically acceptable excipient or carried.

A method for the treatment of a cancer involving a solid tumour in a warm-blooded animal such as a human, may comprise administering to said animal an effective amount of ZD6474 or a pharmaceutically acceptable salt thereof, before, after or simultaneously with an effective amount of pemetrexed and before, after or simultaneously with an effective amount of ionising radiation, wherein ZD6474 and pemetrexed may each optionally be administered together with a pharmaceutically acceptable excipient or carrier.

A method for the treatment of malignant pleural mesothelioma in a warm-blooded animal such as a human, may comprise administering to said animal an effective amount of ZD6474 or a pharmaceutically acceptable salt thereof, before, after or simultaneously with an effective amount of pemetrexed and before, after or simultaneously with an effective amount of ionising radiation, wherein ZD6474 and pemetrexed may each optionally be administered together with a pharmaceutically acceptable excipient or carrier.

A method for the treatment of non-small cell lung cancer (NSCLC) in a warm-blooded animal such as a human, may comprise administering to said animal an effective amount of ZD6474 or a pharmaceutically acceptable salt thereof, before, after or simultaneously with an effective amount of pemetrexed and before, after or simultaneously with an effective amount of ionising radiation, wherein ZD6474 and pemetrexed may each optionally be administered together with a pharmaceutically acceptable excipient or carrier.

A method for the treatment of small cell lung cancer (SCLC) in a warm-blooded animal such as a human, may comprise administering to said animal an effective amount of ZD6474 or a pharmaceutically acceptable salt thereof, before, after or simultaneously with an effective amount of pemetrexed and before, after or simultaneously with an effective amount of ionising radiation, wherein ZD6474 and pemetrexed may each optionally be administered together with a pharmaceutically acceptable excipient or carrier.

There may be provided the use of ZD6474 or a pharmaceutically acceptable salt thereof and pemetrexed in the manufacture of a medicament for use in the production of an antiangiogenic and/or vascular permeability reducing effect in a warm-blooded animal such as a human which is being treated with ionising radiation.

According to the present invention there is provided the use of ZD6474 or a pharmaceutically acceptable salt thereof and pemetrexed in the manufacture of a medicament for use in the production of an anti-cancer effect in a warm-blooded animal such as a human which is being treated with ionising radiation.

According to a further aspect of the present invention there is provided the use of ZD6474 or a pharmaceutically acceptable salt thereof and pemetrexed in the manufacture of a medicament for use in the production of an anti-tumour effect in a warm-blooded animal such as a human which is being treated with ionising radiation.

According to a further aspect of the present invention there is provided the use of ZD6474 or a pharmaceutically acceptable salt thereof and pemetrexed in the manufacture of a medicament for use in the production of an anti-cancer effect in a warm-blooded animal such as a human which is being treated with ionising radiation wherein the cancer is malignant pleural mesothelioma.

According to a further aspect of the present invention there is provided the use of ZD6474 or a pharmaceutically acceptable salt thereof and pemetrexed in the manufacture of a medicament for use in the production of an anti-tumour effect in a warm-blooded animal such as a human which is being treated with ionising radiation wherein the tumour is a malignant pleural mesothelioma.

According to a further aspect of the present invention there is provided the use of ZD6474 or a pharmaceutically acceptable salt thereof and pemetrexed in the manufacture of a medicament for use in the production of an anti-cancer effect in a warm-blooded animal such as a human which is being treated with ionising radiation wherein the cancer is non-small cell lung cancer (NSCLC).

According to a further aspect of the present invention there is provided the use of ZD6474 or a pharmaceutically acceptable salt thereof and pemetrexed in the manufacture of a medicament for use in the production of an anti-tumour effect in a warm-blooded animal such as a human which is being treated with ionising radiation wherein the tumour is a non-small cell tumour of the lung.

According to a further aspect of the present invention there is provided the use of ZD6474 or a pharmaceutically acceptable salt thereof and pemetrexed in the manufacture of a medicament for use in the production of an anti-cancer effect in a warm-blooded animal such as a human which is being treated with ionising radiation wherein the cancer is small cell lung cancer (SCLC).

According to a further aspect of the present invention there is provided the use of ZD6474 or a pharmaceutically acceptable salt thereof and pemetrexed in the manufacture of a medicament for use in the production of an anti-tumour effect in a warm-blooded animal such as a human which is being treated with ionising radiation wherein the tumour is a small cell tumour of the lung.

According to a further aspect of the present invention there is provided a therapeutic combination treatment comprising the administration of an effective amount of ZD6474 or a pharmaceutically acceptable salt thereof, optionally together with a pharmaceutically acceptable excipient or carrier, and the administration of an effective amount of pemetrexed, optionally together with a pharmaceutically acceptable excipient or carrier and the administration of an effective amount of ionising radiation, to a warm-blooded animal such as a human in need of such therapeutic treatment wherein the ZD6474, pemetrexed and ionising radiation may be administered simultaneously, sequentially or separately and in any order.

A warm-blooded animal such as a human which is being treated with ionising radiation means a warm-blooded animal such as a human which is treated with ionising radiation before, after or at the same time as the administration of a medicament or combination treatment comprising ZD6474 and pemetrexed. For example said ionising radiation may be given to said warm-blooded animal such as a human within the period of a week before to a week after the administration of a medicament or combination treatment comprising ZD6474 and pemetrexed. This means that ZD6474, pemetrexed and ionising radiation may be administered separately or sequentially in any order, or may be administered simultaneously. The warm-blooded animal may experience the effect of each of ZD6474, pemetrexed and radiation simultaneously.

According to one aspect of the present invention the ionising radiation is administered before one of ZD6474 and pemetrexed or after one of ZD6474 and pemetrexed.

According to one aspect of the present invention the ionising radiation is administered before both ZD6474 and pemetrexed or after both ZD6474 and pemetrexed.

According to one aspect of the present invention ZD6474 is administered to a warm-blooded animal after the animal has been treated with ionising radiation.

According to another aspect of the present invention the effect of a combination treatment of the present invention is expected to be at least equivalent to the addition of the effects of each of the components of said treatment used alone, that is, of each of ZD6474 and pemetrexed used alone or of each of ZD6474, pemetrexed and ionising radiation used alone.

According to another aspect of the present invention the effect of a combination treatment of the present invention is expected to be greater than the addition of the effects of each of the components of said treatment used alone, that is, of each of ZD6474 and pemetrexed used alone or of each of ZD6474, pemetrexed and ionising radiation used alone.

According to another aspect of the present invention the effect of a combination treatment of the present invention is expected to be a synergistic effect.

According to the present invention a combination treatment is defined as affording a synergistic effect if the effect is therapeutically superior, as measured by, for example, the extent of the response, the response rate, the time to disease progression or the survival period, to that achievable on dosing one or other of the components of the combination treatment at its conventional dose. For example, the effect of the combination treatment is synergistic if the effect is therapeutically superior to the effect achievable with ZD6474 or pemetrexed or ionising radiation alone. Further, the effect of the combination treatment is synergistic if a beneficial effect is obtained in a group of patients that does not respond (or responds poorly) to ZD6474 or pemetrexed or ionising radiation alone. In addition, the effect of the combination treatment is defined as affording a synergistic effect if one of the components is dosed at its conventional dose and the other component(s) is/are dosed at a reduced dose and the therapeutic effect, as measured by, for example, the extent of the response, the response rate, the time to disease progression or the survival period, is equivalent to that achievable on dosing conventional amounts of the components of the combination treatment. In particular, synergy is deemed to be present if the conventional dose of ZD6474 or pemetrexed or ionising radiation may be reduced without detriment to one or more of the extent of the response, the response rate, the time to disease progression and survival data, in particular without detriment to the duration of the response, but with fewer and/or less troublesome side-effects than those that occur when conventional doses of each component are used.

As stated above the combination treatments of the present invention as defined herein are of interest for their antiangiogenic and/or vascular permeability effects. Angiogenesis and/or an increase in vascular permeability is present in a wide range of disease states including cancer (including leukaemia, multiple myeloma and lymphoma), diabetes, psoriasis, rheumatoid arthritis, Kaposi's sarcoma, haemangioma, acute and chronic nephropathies, atheroma, arterial restenosis, autoimmune diseases, acute inflammation, asthma, lymphoedema, endometriosis, dysfunctional uterine bleeding and ocular diseases with retinal vessel proliferation including age-related macular degeneration.

Combination treatments of the present invention are expected to be particularly useful in the prophylaxis and treatment of diseases such as cancer and Kaposi's sarcoma. In particular such combination treatments of the invention are expected to slow advantageously the growth of primary and recurrent solid tumours of, for example, the colon, pancreas, brain, bladder, ovary, breast, prostate, lungs and skin. Combination treatments of the present invention are expected to slow advantageously the growth of tumours in pancreatic cancer, bladder cancer, ovarian cancer, breast cancer and lung cancer, including malignant pleural mesothelioma, small cell lung cancer (SCLC) and non-small cell lung cancer (NSCLC). More particularly such combination treatments of the invention are expected to inhibit any form of cancer associated with VEGF including leukaemia, multiple myeloma and lymphoma and also, for example, to inhibit the growth of those primary and recurrent solid tumours which are associated with VEGF, especially those tumours which are significantly dependent on VEGF for their growth and spread, including for example, certain tumours of the colon (including rectum), pancreas, brain, bladder, ovary, breast, prostate, lung, vulva, skin and particularly malignant pleural mesothelioma and NSCLC. More especially combination treatments of the present invention are expected to slow advantageously the growth of tumours in malignant pleural mesothelioma. More especially combination treatments of the present invention are expected to slow advantageously the growth of tumours in non-small cell lung cancer (NSCLC).

In another aspect of the present invention ZD6474 and pemetrexed, optionally with ionising radiation, are expected to inhibit the growth of those primary and recurrent solid tumours which are associated with VEGF especially those tumours which are significantly dependent on VEGF for their growth and spread.

In another aspect of the present invention ZD6474 and pemetrexed, optionally with ionising radiation, are expected to inhibit the growth of those primary and recurrent solid tumours which are associated with both VEGF and EGF especially those tumours which are significantly dependent on VEGF and EGF for their growth and spread.

The compositions described herein may be in a form suitable for oral administration, for example as a tablet or capsule, for nasal administration or administration by inhalation, for example as a powder or solution, for parenteral injection (including intravenous, subcutaneous, intramuscular, intravascular or infusion) for example as a sterile solution, suspension or emulsion, for topical administration for example as an ointment or cream, for rectal administration for example as a suppository or the route of administration may be by direct injection into the tumour or by regional delivery or by local delivery. In other embodiments of the present invention the ZD6474 of the combination treatment may be delivered endoscopically, intratracheally, intralesionally, percutaneously, intravenously, subcutaneously, intraperitoneally or intratumourally. Preferably ZD6474 is administered orally. In general the compositions described herein may be prepared in a conventional manner using conventional excipients. The compositions of the present invention are advantageously presented in unit dosage form.

ZD6474 will normally be administered to a warm-blooded animal at a unit dose within the range 10-500mg per square metre body area of the animal, for example approximately 0.3-15mg/kg in a human. A unit dose in the range, for example, 0.3-15mg/kg, preferably 0.5-5mg/kg is envisaged and this is normally a therapeutically-effective dose. A unit dosage form such as a tablet or capsule will usually contain, for example 25-500mg of active ingredient. Preferably a daily dose in the range of 0.5-5mg/kg is employed.

Pemetrexed may be administered according to known clinical practice.

For example in NSCLC the recommended dose of pemetrexed is 500mg/m² given by 10 minute intravenous infusion administered on the first day of each 21-day cycle.

For example in malignant pleural mesothelioma the recommended dose of pemetrexed is 500mg/m² given by 10 minute intravenous infusion administered on the first day of each 21-day cycle. When given as well, cisplatin may be administered at 75 mg/m² on Day 1 as a 2-hour intravenous infusion approximately 30 minutes after completion of the administration of pemetrexed.

The dosages and schedules may vary according to the particular disease state and the overall condition of the patient. Dosages and schedules may also vary if, in addition to a combination treatment of the present invention, one or more additional chemotherapeutic agents is/are used. Scheduling can be determined by the practitioner who is treating any particular patient.

Radiotherapy may be administered according to the known practices in clinical radiotherapy. The dosages of ionising radiation will be those known for use in clinical radiotherapy. The radiation therapy used will include for example the use of γ-rays, X-rays, and/or the directed delivery of radiation from radioisotopes. Other forms of DNA damaging factors are also included in the present invention such as microwaves and UV-irradiation. For example X-rays may be dosed in daily doses of 1.8-2.0Gy, 5 days a week for 5-6 weeks. Normally a total fractionated dose will lie in the range 45-60Gy. Single larger doses, for example 5-10Gy may be administered as part of a course of radiotherapy. Single doses may be administered intraoperatively. Hyperfractionated radiotherapy may be used whereby small doses of X-rays are administered regularly over a period of time, for example 0.1Gy per hour over a number of days. Dosage ranges for radioisotopes vary widely, and depend on the half-life of the isotope, the strength and type of radiation emitted, and on the uptake by cells.

The size of the dose of each therapy which is required for the therapeutic or prophylactic treatment of a particular disease state will necessarily be varied depending on the host treated, the route of administration and the severity of the illness being treated. Accordingly the optimum dosage may be determined by the practitioner who is treating any particular patient. For example, it may be necessary or desirable to reduce the above-mentioned doses of the components of the combination treatments in order to reduce toxicity.

The present invention relates to combinations of pemetrexed with ZD6474 or with a salt of ZD6474.

Salts of ZD6474 for use in pharmaceutical compositions will be pharmaceutically acceptable salts, but other salts may be useful in the production of ZD6474 and its pharmaceutically acceptable salts. Such salts may be formed with an inorganic or organic base which affords a pharmaceutically acceptable cation. Such salts with inorganic or organic bases include for example an alkali metal salt, such as a sodium or potassium salt, an alkaline earth metal salt such as a calcium or magnesium salt, an ammonium salt or for example a salt with methylamine, dimethylamine, trimethylamine, piperidine, morpholine or tris-(2-hydroxyethyl)amine.

ZD6474 may be synthesised according to any of the known processes for making ZD6474. For example ZD6474 may be made according to any of the processes described in WO 01/32651; for example those described in Examples 2(a), 2(b) and 2(c) of WO 01/32651.

Pemetrexed is commercially available.

The following tests may be used to demonstrate the activity of ZD6474 in combination with pemetrexed.

### MX-1 human breast cancer xenograft model

Tumour implantation procedures were performed on mice of at least 5 weeks of age. Human tumour xenografts were grown in female athymic (*nu*/*nu* genotype, Swiss) mice. MX-1 tumour fragments were implanted into athymic mice and allowed to grow to 0.7-1cm³ to provide donor tumour tissue. The donor tumours were surgically excised and smaller tumour fragments (20-30 mg) were implanted subcutaneously (s.c.) in the right flanks of the experimental athymic mice. When the mean tumour volume reached 0.1-0.3cm³, randomisation was carried out. Animals were treated with pemetrexed (75mg/kg, intraperitoneally (i.p.) once daily: days 1-5 and 8-12), or with ZD6474 (12.5mg/kg or 25mg/kg,) or drug vehicle, which were administered once-daily orally (p.o.) for the duration of the study (commencing on day 1). An additional group of animals received a combination of pemetrexed and ZD6474, using the same doses and schedules as used for single agent treatment.

Tumour volumes were assessed at least twice weekly by bilateral Vernier caliper measurement. Growth inhibition from the start of treatment was assessed by comparison of the differences in tumour volume between control and treated groups. The effects of combination treatment were assessed by comparing tumour growth in the group of animals receiving pemetrexed plus ZD6474 with the tumour growth in the groups where animals received single agent therapy alone.

An analogous experiment may be used to look at the combination of ZD6474 and pemetrexed with ionising radiation.

### Example 1

Experiments were conducted on female athymic mice (Swiss *nu*/*nu* genotype, ≥6 weeks of age). MX-1 human tumour xenografts were established in mice from tumour cell implant. The donor tumours were surgically excised when they reached a volume of 0.7-1cm³, divided into fragments and frozen until further use. At the start of the experiment, fragments (approximately 30mg) were thawed and implanted (subcutaneously in the dorsal flank) in the experimental animals. Tumour volumes were assessed at least twice weekly by bilateral Vernier calliper measurements. Mice were randomised into treatment groups when the tumour volume reached 0.1-0.2cm³. Following randomisation on day 14 after tumour implantation, mice were treated with either drug vehicle (Control) or ZD6474 (25mg/kg/day) administered orally (p.o.) once daily until end of the study, or with pemetrexed (75mg/kg, intraperitoneally (i.p.) once daily on days 14-18 and 21-25). An additional group of animals received a combination of ZD6474 and pemetrexed, using the same doses and schedules as used for single agent treatment.

Tumour growth inhibition (TGI) from the start of treatment was assessed by comparison of the differences in tumour volume between control and treated groups. The effects of combination treatment were assessed by comparing any effect on tumour growth
in the group of animals receiving pemetrexed plus ZD6474 with tumour growth in the groups where animals received single agent therapy alone.

**Table 1. The effect of pemetrexed or ZD6474 alone and in combination on the growth of NR-1 tumour xenografts in female nude mice.**

| **Group** | **Treatment** | **Day 14 Median tumour volume (cm³)** | **Day 28 Median tumour volume (cm³)** | **Day 14 geometric mean tumour volume (cm³)** | **Day 28 geometric mean tumour volume (cm³)** |
|---|---|---|---|---|---|
| GROUP 1 | Control | 0.136 | 1.368 | 0.148 | 1.520 |
| GROUP 3 | ZD6474 (25mg/kg) | 0.148 | 0.967 | 0.148 | 0.702 |
| GROUP 4 | pemetrexed (75mg/kg: Day 0-4 & Days 7-11) | 0.147 | 0.794 | 0.146 | 0.751 |
| GROUP 6 | ZD6474 (25mg/kg) + pemetrexed (75mg/kg: Day 0-4 & Days 7-11) | 0.133 | 0.566 | 0.146 | 0.610 |

Median and mean tumour volumes were lower in tumour-bearing nude mice treated with ZD6474 (25mg/kg/day) and pemetrexed (75mg/kg/day, Days 14-18 & Days 21-25) compared with animals treated with each of ZD6474 (25mg/kg/day) or pemetrexed (75mg/kg/day, Days 14-18 & Days 21-25) alone.

An analogous experiment may be used to look at the combination of ZD6474 and pemetrexed with ionising radiation.

## Claims

1. Use of ZD6474 or a pharmaceutically acceptable salt thereof and pemetrexed in the manufacture of a medicament for use in the production of an anti-cancer effect in a warm-blooded animal such as a human.

2. Use of ZD6474 or a pharmaceutically acceptable salt thereof and pemetrexed in the manufacture of a medicament for use in the production of an anti-tumour effect in a warm-blooded animal such as a human.

3. Use according to claim 1 wherein the warm-blooded animal such as a human is being treated with ionising radiation.

4. Use according to claim 2 wherein the warm-blooded animal such as a human is being treated with ionising radiation

5. Use according to claim 2 or claim 4 wherein the tumour is malignant pleural mesothelioma or is a non-small cell tumour of the lung or is a small cell tumour of the lung.

6. Use according to claim 1 or claim 3 wherein the cancer is non-small cell lung cancer (NSCLC) or malignant pleural mesothelioma or small cell lung cancer (SCLC).

7. A pharmaceutical composition which comprises ZD6474 or a pharmaceutically acceptable salt thereof, and pemetrexed in association with a pharmaceutically acceptable excipient or carrier.

8. A kit comprising ZD6474 or a pharmaceutically acceptable salt thereof and pemetrexed.

## Patentansprüche

1. Verwendung von ZD6474 oder einem pharmazeutisch annehmbaren Salz davon und Pemetrexed bei der Herstellung eines Arzneimittels zur Erzielung einer Antikrebswirkung bei einem Warmblüter wie einem Menschen.

2. Verwendung von ZD6474 oder einem pharmazeutisch annehmbaren Salz dvon und Pemetrexed bei der Herstellung eines Arzneimittels zur Erzielung einer Antitumorwirkung bei einem Warmblüter wie einem Menschen.

3. Verwendung nach Anspruch 1, wobei der Warmblüter wie ein Mensch mit ionisierender Strahlung behandelt wird.

4. Verwendung nach Anspruch 2, wobei der Warmblüter wie ein Mensch mit ionisierender Strahlung behandelt wird.

5. Verwendung nach Anspruch 2 oder Anspruch 4, wobei es sich bei dem Tumor um malignes Pleuramesotheliom oder einen nicht-kleinzelligen Tumor der Lunge oder einen kleinzelligen Tumor der Lunge handelt.

6. Verwendung nach Anspruch 1 oder Anspruch 3, wobei es sich bei dem Krebs um nicht-kleinzelliges Bronchialkarzinom (NSCLC) oder malignes Pleuramesotheliom oder kleinzelliges Bronchialkarzinom (SCLC) handelt.

7. Pharmazeutische Zusammensetzung, die ZD6474 oder ein pharmazeutisch annehmbares Salz davon und Pemetrexed zusammen mit einem pharmazeutisch annehmbaren Hilfsstoff oder Träger enthält.

8. Kit, enthaltend ZD6474 oder ein pharmazeutisch annehmbares Salz davon und Pemetrexed.

## Revendications

1. Utilisation de ZD6474 ou d'un sel pharmaceutiquement acceptable de celui-ci et de pemetrexed dans la fabrication d'un médicament destiné à être utilisé dans la production d'un effet anticancéreux chez un animal à sang chaud tel que l'homme.

2. Utilisation de ZD6474 ou d'un sel pharmaceutiquement acceptable de celui-ci et de pemetrexed dans la fabrication d'un médicament destiné à être utilisé dans la production d'un effet antitumoral chez un animal à sang chaud tel que l'homme.

3. Utilisation selon la revendication 1, **caractérisée en ce que** l'animal à sang chaud tel que l'homme reçoit un traitement par radiations ionisantes.

4. Utilisation selon la revendication 2, **caractérisée en ce que** l'animal à sang chaud tel que l'homme reçoit un traitement par radiations ionisantes.

5. Utilisation selon la revendication 2 ou la revendication 4, **caractérisée en ce que** la tumeur est le mésothéliome pleural malin ou est une tumeur non à petites cellules du poumon ou est une tumeur à petites cellules du poumon.

6. Utilisation selon la revendication 1 ou la revendication 3, **caractérisée en ce que** le cancer est le cancer du poumon non à petites cellules (NSCLC) ou le mésothéliome pleural malin ou le cancer du poumon à petites cellules (SCLC).

7. Composition pharmaceutique qui comprend le ZD6474 ou un sel pharmaceutiquement acceptable de celui-ci, et le pemetrexed en association avec un excipient ou un support pharmaceutiquement acceptable.

8. Kit comprenant le ZD6474 ou un sel pharmaceutiquement acceptable de celui-ci et le pemetrexed.
